# FASCICULE DE BREVET EUROPEEN

(11) **EP 2 522 823 B1**
(45) Date de publication et mention de la délivrance du brevet: **23.04.2014**
(21) Numéro de dépôt: 11003978.1
(22) Date de dépôt: 13.05.2011
(51) Int. Cl.: F01N 3/20, F17C 11/00, G05D 9/00, G05D 15/00

(54) **Dispositif de mesure d'une quantité d'un agent réducteur, de préférence de NH3, contenu dans un réservoir**
Vorrichtung zur Messung der Menge eines in einem Behälter enthaltenen Reduktionsmittels, vorzugsweise NH3
Device for measuring an amount of a reducing agent, preferably NH3, contained in a tank

(43) Date de publication de la demande: 14.11.2012
(73) Titulaire: Aaqius & Aaqius S.A., 1207 Genève (CH)
(72) Inventeur: Audouin, Arnaud, 75018 Paris (FR)
(74) Mandataire: Regimbeau

(56) Documents cités:
- EP-A1- 1 977 817
- WO-A2-2006/012903
- DE-A1-102009 022 884
- US-A- 4 600 525
- US-A1- 2006 184 307

## Description

L'invention proposée concerne un dispositif de mesure du niveau d'un agent réducteur contenu dans un réservoir. L'invention porte également sur un procédé de régulation d'injection de l'agent réducteur dans les gaz d'échappement d'un véhicule à moteur.

Les émissions de polluants liées au transport sont depuis près de trente ans un moteur de progrès de premier plan de l'industrie. La sévérisation progressive des limites en émission pour les quatre polluants réglementés (CO, HC, NOx, particules) a permis d'améliorer significativement la qualité de l'air notamment dans les grandes agglomérations.

L'utilisation toujours croissante de l'automobile nécessite de poursuivre les efforts pour réduire encore davantage ces émissions de polluants. Ainsi, la réduction des oxydes d'azote (NOx) reste une problématique complexe dans le contexte de la sévérisation des seuils émission Européen attendu en 2015 pour l'entrée en vigueur de la norme €6. Disposer de technologies de dépollution à forte efficacité dans toutes les conditions de roulage reste un enjeu majeur pour l'industrie du transport.

Dans un deuxième temps, la consommation de carburant, en lien direct avec les émissions de CO2, est devenue une préoccupation majeure. Ainsi, une réglementation sera mise en place au niveau Européen à partir de 2012 sur les émissions de CO2 des véhicules particuliers. Il est d'ores et déjà acquis que cette limite sera régulièrement abaissée au cours des décennies à venir. La réduction de CO2 s'est donc imposée à l'évidence pour toute l'industrie des transports.

Cette double problématique réduction de la pollution locale (NOx) et réduction de la consommation carburant (CO2) est particulièrement difficile pour le moteur Diesel dont la combustion en mélange pauvre s'accompagne d'émission NOx difficile à traiter.

Il existe déjà des dispositifs tels que celui décrit dans EP1977817 permettant la réduction de la quantité de NOx au moyen d'un catalyseur SCR *(Selective Catalytic Réduction)* utilisant de l'ammoniac stocké dans un matériau de stockage du type sels chlorures d'alcalino-terreux agencé à l'intérieur d'un réservoir. L'injection d'ammoniac dans les gaz d'échappements est pilotée grâce à un dispositif de chauffage permettant de chauffer le matériau de stockage afin de permettre la réaction réversible d'absortion/désorption de l'ammoniac puisque cette réaction est directement liée à la température au sein du matériau de stockage.

En pratique, l'ammoniac est injecté à l'échappement en continu dans les proportions stoechiométriques de la réaction de réduction des NOx. Il convient donc de pouvoir stocker à bord une quantité suffisante d'ammoniac. Pour limiter l'encombrement du réservoir contenant le matériau de stockage, les constructeurs automobiles privilégient un remplissage ou un remplacement du réservoir périodiquement, par exemple lors de la maintenance moteur (vidange) ou lors d'un remplissage réservoir carburant. Selon les véhicules considérés (véhicules particuliers, poids lourds...) il est nécessaire de prévoir entre 10 et 100 opérations de remplissage du réservoir ou de remplacement de celui-ci pendant la durée de vie du véhicule.

Cette opération de maintenance périodique, nécessaire pour assurer une dépollution efficace des NOx pendant toute la vie du véhicule, fait l'objet de réglementations spécifiques dans les différents pays ou la technologie SCR est utilisée. Un point commun à toutes ces réglementations est la nécessité de pouvoir déterminer la quantité d'ammoniac restant dans le réservoir pour pouvoir avertir le conducteur lorsqu'un remplissage doit être fait. Par exemple, dans la législation Européenne pour les véhicules particuliers, il est nécessaire de pouvoir mesurer au minimum deux seuils d'autonomie restante, à 2400 km et 800km (correspondant approximativement à respectivement 3 pleins et 1 plein de carburant).

Par ailleurs, dans le cas où plusieurs réservoirs comportant chacun un matériau de stockage de l'ammoniac sont embarqués dans le véhicule afin de simplifier l'intégration du système de stockage d'ammoniac dans le véhicule, ou pour en améliorer son fonctionnement (introduction d'une unité à froid), il est nécessaire de connaître la quantité restante d'ammoniac dans chaque réservoir afin que le calculateur moteur puisse piloter de manière optimale l'injection de l'ammoniac contenu dans ces différents réservoir.

Le document DE 10 2009 022884 A1 décrit de son côté un dispositif de mesure de la quantité d'ammoniac basé sur la variation de capacité d'un condensateur électrique.

Le document US 2006/184307 A1 décrit un procédé de régulation d'injection d'un agent réducteur par comparaison de la consommation moyenne avec une valeur seuil pour limiter, le cas échéant, cette consommation.

Ainsi, le but de la présente invention est de proposer un dispositif de mesure du niveau d'un agent réducteur, de préférence de l'ammoniac, contenu dans un réservoir.

Conformément à l'invention ce but est atteint grâce un dispositif de mesure d'une quantité d'un agent réducteur, de préférence de NH₃, contenu dans un réservoir renfermant un réseau cristallin (ci-après matériau de stockage) dans lequel est stocké l'agent réducteur, le volume du matériau de stockage variant en fonction de la quantité de l'agent réducteur qu'il contient. Le dispositif de mesure comporte des moyens associés au réservoir, lesdits moyens étant adaptés pour mesurer la quantité de l'agent réducteur stockée dans le matériau de stockage en fonction du volume de ce dernier. De plus ces moyens sont agencés d'une part pour mesurer une contrainte mécanique générée par la dilatation du matériau de stockage, et d'autre part pour convertir l'information de contrainte mesurée en une quantité de l'agent réducteur stockée dans le matériau de stockage.

Un autre aspect de l'invention porte sur un procédé de régulation d'injection d'un agent réducteur dans les gaz d'échappement d'un véhicule à moteur comprenant au moins les étapes suivantes:
- mesure de la quantité de l'agent réducteur dans un réservoir alimentant le moteur avec le dispositif de mesure selon l'invention.
- déterminer la consommation moyenne de l'agent réducteur depuis la dernière opération de maintenance du réservoir,
- comparer ladite consommation moyenne à au moins une valeur seuil afin de limiter, cas échéant, cette consommation de manière à disposer d'une quantité suffisante d'agent réducteur pour atteindre le pas théorique de la prochaine opération de maintenance du réservoir.

Les caractéristiques de l'invention apparaitront plus clairement à la lecture de la description de plusieurs variantes d'exécution, données uniquement à titre d'exemples, nullement limitatives en se référant aux figures schématiques dans lesquelles:
- La Figure 1 représente les étapes essentielles dans la préparation du matériau de stockage;
- La figure 2 illustre le réservoir comportant le matériau de stockage respectivement chargé et déchargé en ammoniac selon une première configuration;
- La figure 3 illustre le réservoir comportant le matériau de stockage respectivement chargé et déchargé en ammoniac selon une deuxième configuration;
- La figure 4 illustre le réservoir comportant le matériau de stockage respectivement chargé et déchargé en ammoniac selon une troisième configuration;
- La figure 5 illustre l'évolution de la tension mesurée par les moyens de mesure de contrainte mécanique en fonction du taux de remplissage d'ammoniac dans le réservoir;
- La figure 6 représente une cartographie pour la détermination du niveau de limitation de consommation d'ammoniac;
- La figure 7 représente une cartographie de correction sur la quantité d'ammoniac à injecter;
- La figure 8 représente l'évolution de la quantité d'ammoniac consommée en fonction de la distance parcourue par le véhicule à moteur pour des conditions de roulage dite moyennes;
- La Figure 9 représente l'évolution de la quantité d'ammoniac consommée qui a été limitée pour des conditions de roulage dite sévères.

Les différents modes d'exécution selon l'invention qui sont décrit ci-après reposent sur le fait qu'au cours de la réaction d'absorption, la fixation de l'ammoniac dans un sel de type chlorure métallique, s'accompagne d'une augmentation de volume, l'ammoniacate occupant alors un volume très supérieur au volume du sel pur (jusqu'à un rapport de 4 à 5 fois). L'augmentation de volume du sel est due non seulement à la dilatation de son réseau cristallin mais aussi à son fractionnement laissant ainsi de l'espace libre entre les microcristaux de complexe d'ammociate.

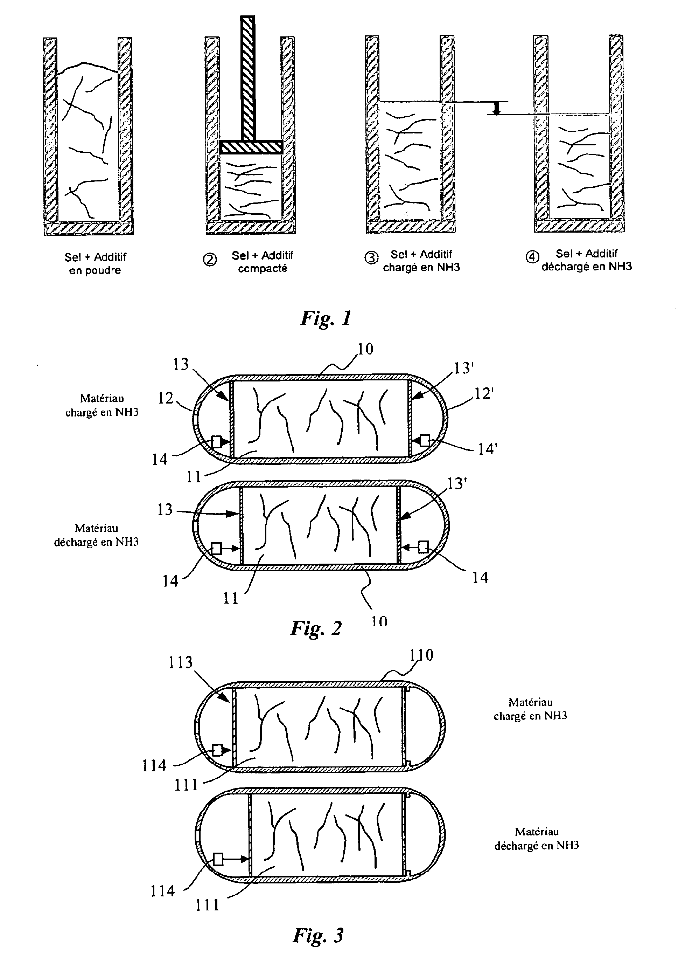

Selon l'invention, le matériau de stockage comporte de préférence du graphite naturel expansé afin que ce matériau possède une meilleure conductivité thermique d'une part, et qu'il soit plus robuste d'autre part. Selon la préparation de ce matériau de stockage telle qu'illustrée par la Figure 1, le graphite naturel expansé est ajouté au sel avant d'être compacté selon un axe unidirectionnel. De cette façon, la dilatation qui résulte de la saturation du matériau en ammoniac se fait avant tout selon l'axe de compression, et dans une moindre mesure perpendiculairement à l'axe de compression. Ainsi, il est possible de contrôler la dilatation du matériau selon l'axe de compression, avec pour intérêt de limiter la contrainte appliquée aux parois de l'enceinte de stockage.

Ensuite lors de la désorption de l'ammoniac, le réseau créé par le graphite naturel expansé recompressé forme une structure robuste qui maintient les particules de sels déchargées en ammoniac. Selon la qualité de mise en oeuvre, on observe une dilatation comprise entre 1 et 10% selon l'axe de compression et de l'ordre de 0.01 et 1% selon l'axe perpendiculaire à l'axe de compression.

Selon un premier mode d'exécution tel qu'illustré à la Figure 2, le réservoir 10 contenant le matériau de stockage 11 est à symétrie de révolution, préférentiellement cylindrique, ce réservoir 10 étant fermé à ces deux extrémités par deux demi-sphères 12, 12'. Un premier et un second disque 13, 13' perméables aux gaz d'ammoniac sont agencés de part et d'autre du matériau de stockage 11 et sont susceptibles de se déplacer selon l'axe de compression dudit matériau 11. Un premier et un second capteur piézoélectrique 14, 14' sont positionnés respectivement dans l'une et l'autre des demi-sphères 12, 12' de manière à être en contact avec le premier respectivement le second disque 13, 13' afin de mesurer la dilatation du matériau de stockage 11 selon son axe de compression au cours de la désorption de l'ammoniac.

Selon un second mode d'exécution de l'invention tel qu'illustré à la Figure 3, un premier et un second disque 113, 113' perméables aux gaz d'ammoniac sont agencés de part et d'autre du matériau de stockage 111 à l'intérieur du réservoir 110. Le premier disque 113 est susceptible de se déplacer selon l'axe de compression du matériau 111 alors que le second disque 113' est maintenu immobile. Un capteur piézoélectrique 114 est positionné de manière à être en contact avec le premier disque 113 pour pouvoir mesurer la dilatation du matériau de stockage 111 selon son axe de compression au cours de la désorption de l'ammoniac.

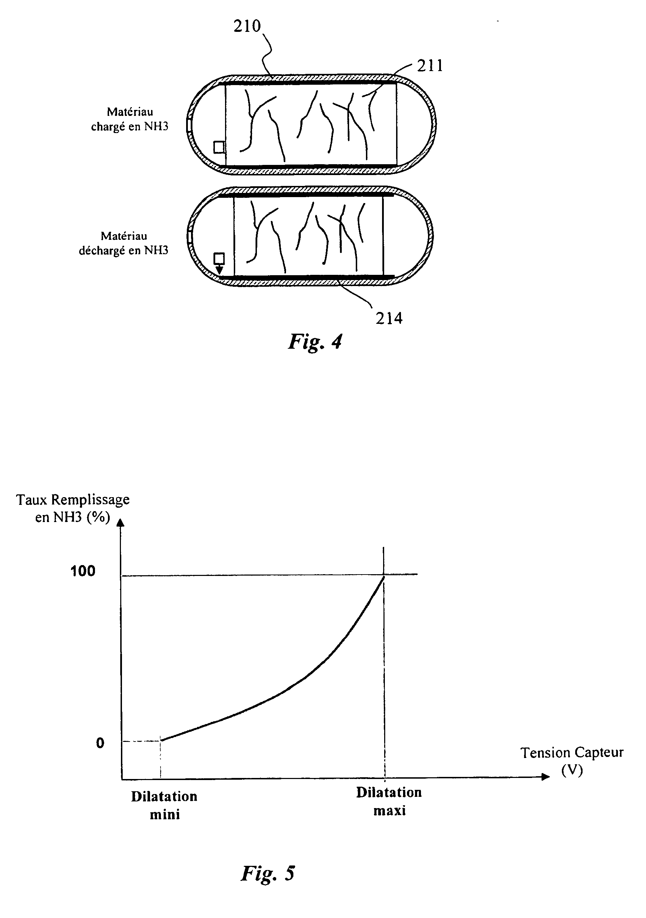

Selon un troisième mode d'exécution de l'invention tel qu'illustré à la Figure 4, c'est la dilatation qui s'effectue perpendiculairement à l'axe de compression du matériau de stockage 211 qui est mesurée. Dans ce cas, un matériau piézoélectrique 214 couvrant toute la surface externe du matériau de stockage 211 est positionné entre le réservoir 210 et le matériau de stockage 211. A mesure que le matériau de stockage 211 se vide en ammoniac, la contrainte mécanique imposée au matériau piézoélectrique 214 diminue.

Précisons que tout autre moyen apte à mesurer la dilation du matériau de stockage peut être utilisé à la place d'un ou des plusieurs capteurs piézoélectriques.

Pour ces trois modes d'exécution de l'invention, l'évolution de la contrainte mécanique imposée par la dilatation du matériau de stockage au cours d'un cycle complet de désorption peut être directement corrélée avec l'ammoniac présent dans le réservoir. Ainsi, il est possible de définir par calibration pour le réservoir considéré le niveau d'ammoniac restant par mesure de la tension aux bornes du matériau piézoélectrique comme schématisé sur la Figure 5.

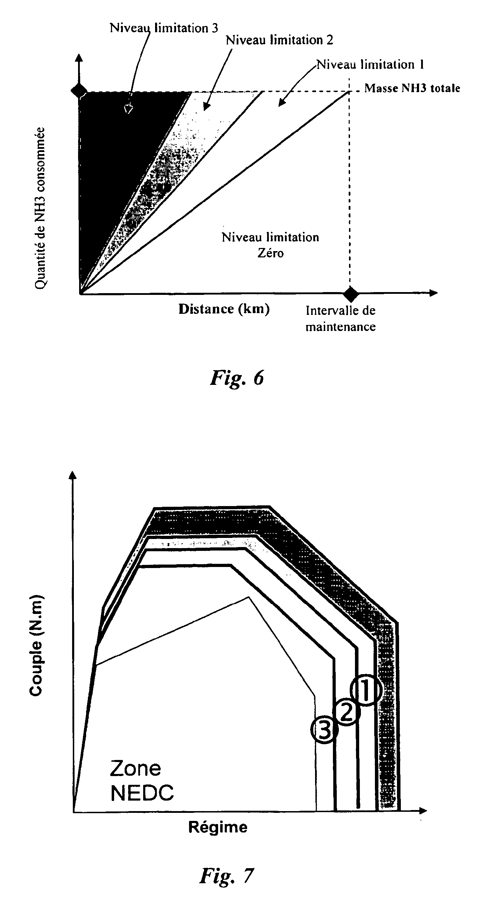

Une opération de maintenance périodique, calée préférentiellement sur la maintenance moteur (vidange) ou sur un remplissage carburant, est réalisée pour remplir en ammoniac le réservoir comportant le matériau de stockage selon l'invention. Le réservoir vide en ammoniac peut aussi être remplacé par un nouveau réservoir dont le matériau de stockage est saturé en ammoniac. Le jaugeage de l'ammoniac restant décrit ci-dessus associé à l'acquisition de l'opération de maintenance permet selon l'invention de déterminer par calcul une consommation moyenne d'ammoniac depuis la dernière opération de maintenance. Dans la présente invention, cette consommation moyenne mesurée est comparée à diverses valeurs seuils dans le but d'adapter les stratégies de consommation pour, le cas échéant, limiter cette consommation et ainsi s'assurer une autonomie suffisante en ammoniac pour atteindre le pas de maintenance théorique du réservoir. En pratique, la quantité d'ammoniac consommée depuis la dernière opération de maintenance mesurée via l'utilisation du ou des capteurs piézoélectriques détermine un niveau de limitation au moyen d'une cartographie telle que définie à la Figure 6.

Typiquement, tant que la consommation d'ammoniac reste inférieure au seuil niveau 1, le niveau de limitation zéro est actif (i.e. pas de correction de la quantité d'ammoniac injectée). Si à partir d'un certain kilométrage, la consommation d'ammoniac vient à excéder la courbe seuil niveau 1, le niveau de limitation 1 est activé, les corrections correspondantes sont alors appliquées.

Pour chaque niveau de limitation, une correction est appliquée sur la quantité d'ammoniac à injecter calculée pour les besoins en conversion NOx du dispositif de post-traitement SCR. Ce coefficient de correction, appliqué sous la forme d'un coefficient multiplicatif inférieur à 1, est déterminé pour chaque niveau de limitation, au moyen d'une cartographie régime/charge comme représenté à la Figure 7 où la zone NEDC (New European Driving Cycle) est la zone couverte lors d'un cycle homologation. Selon cette figure, la consommation en ammoniac est limitée progressivement par trois coefficients de correction, dès que le moteur fonctionne respectivement dans la zone 1, puis dans la zone 2, et enfin dans la zone 3. Les paramètres température ambiante, altitude et température d'eau du moteur pourront aussi être pris en compte pour déterminer la valeur du coefficient de correction.

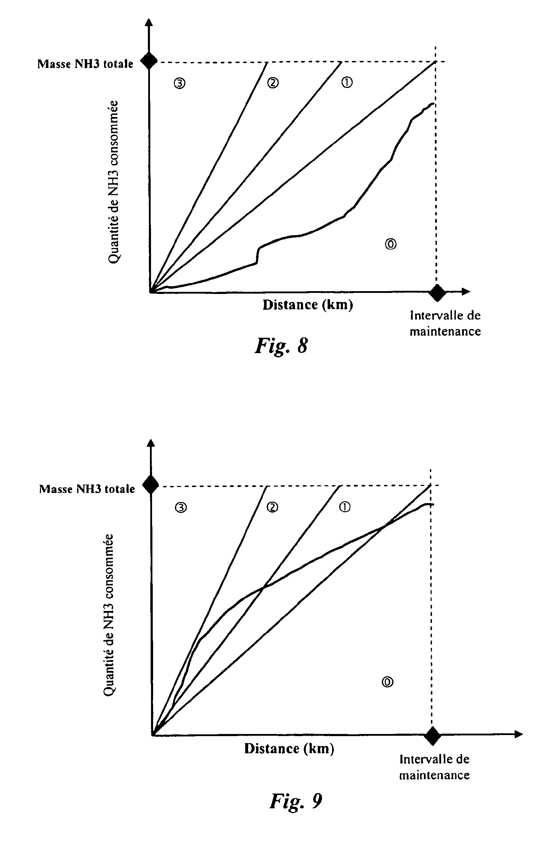

En mettant en place ces niveaux de limitation successif, il devient possible de contrôler la consommation d'ammoniac de façon adapté aux conditions de roulage du chaque client. Etant donnée que la majorité des clients présente des conditions de roulage moyennes, l'injection d'ammoniac n'est pas corrigée et l'efficacité maximale du système est obtenue (Figure 8). Pour les 10% de clients qui présentent des conditions de roulage sévères, l'injection d'ammoniac non corrigée conduirait à consommer la totalité de l'ammoniac stockée dans le réservoir avant l'intervalle de vidange. La mise en place de niveaux de limitation permet de limiter progressivement la consommation d'ammoniac, au prix d'une réduction de l'efficacité du système, et donc d'assurer le fonctionnement du système jusqu'à l'intervalle du vidange (Figure 9).

Il va de soit que l'invention n'est pas limitée aux modes d'exécution décrits ci-dessus à titre d'exemples mais qu'elle embrasse, au contraire toutes les variantes d'exécution. Par exemple, le dispositif de mesure peut aussi être utilisé pour l'estimation de la quantité d'hydrure d'uns pile à combustible d'un système de stockage d'hydrogène.

## Revendications

1. Dispositif de mesure d'une quantité d'un agent réducteur, de préférence de NH₃, contenu dans un réservoir (10; 110; 210) renfermant un matériau de stockage (11; 111; 211) dans lequel est stocké l'agent réducteur, le volume du matériau de stockage (11; 111; 211) variant en fonction de la quantité de l'agent réducteur qu'il contient, le dispositif de mesure comportant des moyens (13, 14, 14'; 113, 114; 213, 214) associés au réservoir (10; 110; 210), lesdits moyens étant adaptés pour mesurer la quantité de l'agent réducteur stockée dans le matériau de stockage (11 ; 111 ; 211) en fonction du volume de ce dernier, **caracterisé en ce que** ces moyens (13, 14, 14'; 113, 114; 213, 214) sont agencés d'une part pour mesurer une contrainte mécanique générée par la dilatation du matériau de stockage (11; 111; 211), et d'autre part pour convertir l'information de contrainte mesurée en une quantité de l'agent réducteur stockée dans le matériau de stockage (11; 111; 211).

2. Dispositif de mesure selon la revendication 1, dans lequel lesdits moyens comportent au moins un capteur piézoélectrique (14,14'; 114, 214).

3. Dispositif de mesure selon la revendication 2, dans lequel lesdits moyens comportent en outre au moins une paroi mobile (13, 13'; 113) en contact avec le matériau de stockage (11; 111; 211), ledit capteur piézoélectrique (14, 14'; 114, 214) étant agencé pour effectuer une mesure de contrainte mécanique lié au déplacement de la paroi mobile (13, 13'; 113).

4. Dispositif de mesure selon l'une quelconque des revendications 1 à 3, dans lequel lesdits moyens comportent deux capteurs piézoélectriques (14, 14') et deux parois mobiles (13, 13'), chaque capteur piézoélectrique (14, 14') étant associé à une paroi mobile (13, 13') respective pour la mesure de la contrainte mécanique lié au déplacement de celle-ci.

5. Dispositif de mesure selon la revendication 3 ou 4, dans lequel au moins une paroi mobile (13, 13'; 113) est perméable à l'agent réducteur.

6. Dispositif de mesure selon l'une quelconque des revendications 3 à 5, dans lequel chaque paroi (13, 13'; 113) est positionnée perpendiculairement à un axe longitudinal du réservoir (10; 110; 210).

7. Dispositif de mesure selon la revendication 1, dans lequel lesdits moyens (214) sont agencés autour de la surface latéral du matériau de stockage (211).

8. Procédé de régulation d'injection d'un agent réducteur dans les gaz d'échappement d'un véhicule à moteur comprenant au moins les étapes suivantes:
- mesure de la quantité de l'agent réducteur dans un réservoir alimentant le moteur, en particulier avec le dispositif de mesure selon l'une quelconque des revendications 1 à 7,
- déterminer la consommation moyenne de l'agent réducteur depuis la dernière opération de maintenance du réservoir,
- comparer ladite consommation moyenne à au moins une valeur seuil afin de limiter, cas échéant, cette consommation de manière à disposer d'une quantité suffisante d'agent réducteur pour atteindre le pas théorique de la prochaine opération de maintenance du réservoir.

9. Procédé selon la revendication 8, dans lequel la consommation de l'agent réducteur est limitée en fonction:
- du régime moteur, la charge motrice et/ou la température d'eau du moteur; de la distance parcourue par le véhicule à moteur depuis la dernière opération de maintenance du réservoir; et/ou
- un paramètre extérieur, en particulier la température ambiante et/ou l'altitude.

## Patentansprüche

1. Messvorrichtung zur Messung der Menge eines Reduktionsmittels, insbesondere von NH₃, das in einem Behälter (10; 110; 210) enthalten ist, welcher ein Speichermaterial (11; 111; 211) umschließt, in dem das Reduktionsmittel gespeichert ist, wobei das Volumen des Speichermaterials (11; 111; 211) in Abhängigkeit von der Menge des Reduktionsmittels, welches es enthält, variiert, wobei die Messvorrichtung Mittel (13, 14, 14'; 113, 114; 213, 214) aufweist, die mit dem Behälter (10; 110; 210) verbunden sind, wobei die Mittel dafür ausgelegt sind, um die Menge des in dem Speichermaterial (11; 111; 211) gespeicherten Reduktionsmittels in Abhängigkeit vom Volumen des Speichermaterials zu messen, **dadurch gekennzeichnet, dass** diese Mittel (13, 14, 14'; 113, 114; 213, 214) zum einen dafür angeordnet sind, um eine mechanische Spannung, die durch die Ausdehnung des Speichermaterials (11; 111; 211) erzeugt wird, zu messen, und zum anderen um die Information der gemessenen Spannung in eine Menge des in dem Speichermaterial (11; 111; 211) gespeicherten Reduktionsmittels zu konvertieren.

2. Messvorrichtung nach Anspruch 1, wobei die Mittel mindestens einen piezoelektrischen Sensor (14, 14', 114, 214) aufweisen.

3. Messvorrichtung nach Anspruch 2, wobei die Mittel mindestens eine bewegliche Wand (13, 13', 113) in Kontakt mit dem Speichermaterial (11; 111; 211) aufweisen, wobei der piezoelektrische Sensor (14, 14'; 114, 214) dafür angeordnet ist, um eine Messung der an die Verschiebung der beweglichen Wand (13, 13'; 113) gebundenen mechanischen Spannung durchzuführen.

4. Messvorrichtung nach einem der Ansprüche 1 bis 3, wobei die Mittel zwei piezoelektrische Sensoren (14, 14') und zwei bewegliche Wände (13, 13') aufweisen, wobei jeder piezoelektrische Sensor (14, 14') mit einer betreffenden mobilen Wand (13, 13') verbunden ist, um deren an die Verschiebung gebundene mechanische Spannung zu messen.

5. Messvorrichtung nach Anspruch 3 oder 4, wobei die mindestens eine bewegliche Wand (13, 13'; 113) für das Reduktionsmittel durchlässig ist.

6. Messvorrichtung nach einem der Ansprüche 3 bis 5, wobei jede Wand (13, 13'; 113) senkrecht zu einer Längsachse des Behälters (10; 110; 210) angeordnet ist.

7. Messvorrichtung nach Anspruch 1, wobei die Mittel (214) um die seitliche Oberfläche des Speichermaterials (211) angeordnet sind.

8. Verfahren zur Steuerung der Einspritzung eines Reduktionsmittels in das Abgas eines Motorfahrzeugs, umfassend mindestens die folgenden Schritte:
- Messen der Menge des Reduktionsmittels in einem Versorgungsbehälter des Motors, insbesondere mittels der Messvorrichtung nach einem der Ansprüche 1 bis 7,
- Bestimmen des mittleren Verbrauchs des Reduktionsmittels seit dem letzten Wartungsvorgang des Behälters,
- Vergleichen des mittleren Verbrauchs mit mindestens einem Schwellenwert, um ggf. diesen Verbrauch zu begrenzen, in der Weise, dass eine ausreichende Menge des Reduktionsmittels disponiert wird, um das theoretische Intervall des nächsten Wartungsvorgangs des Behälters zu erreichen.

9. Verfahren nach Anspruch 8, wobei der Verbrauch des Reduktionsmittels begrenzt wird in Abhängigkeit von:
- der Motorbetriebsart, der Antriebsleistung und/oder der Wassertemperatur des Motors; der von dem Motorfahrzeug seit dem letzten Wartungsvorgang des Behälters zurückgelegten Entfernung; und/oder
- einem externen Parameter, insbesondere der Umgebungstemperatur und/oder der Höhe.

## Claims

1. A device for measuring an amount of a reducing agent, preferably NH₃, contained in a vessel (10; 110; 210) containing a storage material (11; 111; 211) in which the reducing agent is stored, the volume of the storage material (11; 111; 211) varying according to the amount of reducing agent which it contains, the measuring device including means (13, 14, 14'; 113, 114; 213, 214) associated with the vessel (10; 110; 210), said means being suitable for measuring the amount of reducing agent stored in the storage material (11; 111; 211) according to the volume of the latter, **characterized in that** said means (13, 14, 14'; 113, 114; 213, 214) are arranged on the one hand for measuring mechanical stress generated by expansion of the storage material (11; 111; 211), and on the other hand for converting the stress measurement information into an amount of reducing agent stored in the storage material (11; 111; 211).

2. The measuring device according to claim 1, wherein said means include at least one piezoelectric sensor (14, 14'; 114, 214).

3. The measuring device according to claim 2, wherein said means further include at least one mobile wall (13, 13'; 113) in contact with the storage material (11; 111; 211), said piezoelectric sensor (14, 14'; 114, 214) being arranged to measure mechanical stress related to movement of the mobile wall (13, 13'; 113).

4. The measuring device according to any of claims 1 to 3, wherein said means include two piezoelectric sensors (14, 14') and two mobile walls (13, 13'), each piezoelectric sensor (14, 14') being associated with a respective mobile wall (13, 13') so as to measure mechanical stress related to movement thereof.

5. The measuring device according to claim 3 or 4, wherein at least one mobile wall (13, 13'; 113) is permeable to the reducing agent.

6. The measuring device according to any of claims 3 to 5, wherein each wall (13, 13'; 113) is positioned perpendicular to a longitudinal axis of the vessel (10; 110; 210).

7. The measuring device according to claim 1, wherein said means (214) are arranged around the lateral surface of the storage material (211).

8. A method for controlling the injection of a reducing agent into the exhaust gases of a motor vehicle, comprising at least the following steps:
- measuring the amount of reducing agent in a vessel feeding the engine, in particular with the measuring device according to any of claims 1 to 7,
- determining the average consumption of reducing agent since the last vessel maintenance service,
- comparing said average consumption with at least one threshold value so as to, if need be, limit said consumption so as to have a sufficient amount of reducing agent to reach the theoretical step of the next vessel maintenance service.

9. The method according to claim 8, wherein consumption of the reducing agent is limited according to:
- the engine speed, engine load and/or engine water temperature; the distance traveled by the motor vehicle since the last vessel maintenance service; and/or
- an external parameter, in particular ambient temperature and/or altitude.
